# EUROPEAN PATENT SPECIFICATION

(11) **EP 0 728 493 B1**
(45) Date of publication and mention of the grant of the patent: **20.03.2002**
(21) Application number: 95120694.5
(22) Date of filing: 28.12.1995
(51) Int. Cl.: A61M 16/00, A61B 5/083

(54) **Ventilator/anaesthetic system**
Beatmungsgerät/Narkosesystem
Appareil respiratoire/système d'anesthésie

(30) Priority: 27.02.1995 SE 9500713
(43) Date of publication of application: 28.08.1996
(73) Proprietor: Siemens-Elema AB, 171 95 Solna 1 (SE)
(72) Inventor: Olsson, Sven-Gunnar, S-232 00 Arlöv (SE); Rydgren, Göran, S-230 44 Bunkeflostrand (SE); Brauer, Stefan, S-224 73 Lund (SE); Linge, Anders, S-244 36 Kävlinge (SE)

(56) References cited:
- EP-A- 0 392 503
- EP-A- 0 584 519
- US-A- 4 619 269

## Description

The present invention relates to a ventilator/anaesthetic system comprising a ventilator/anaesthetic unit, an inspiratory tube to carry a breathing gas from the ventilator/anaesthetic unit to a patient, an expiratory tube to carry expired breathing gas from the patient to the ventilator/anaesthetic unit, a patient tube, connected to the inspiratory tube and the expiratory tube and connectable to the patient's airways, a flow meter arranged to measure the flow of expired gas, a carbon dioxide meter to measure the concentration of carbon dioxide in expired breathing gas and a calculation unit connected to the carbon dioxide meter and the flow meter to determine at least one parameter related to the patient's carbon dioxide output.

Ventilator systems are normally used for supporting or controlling the respiration of patients with respiratory problems or who are incapable of breathing without aid. The problems can be caused by lung disease or damage to the lungs. Anaesthetic systems are used for inducing anaesthesia in patients about to undergo surgery. With both systems, it is important to obtain some measure on the efficacy of the patient's ventilation. Information on whether the patient's blood is being oxygenated to a sufficient degree is especially important. One highly useful procedure in this context is to study some parameter related to the patient's carbon dioxide output. One such parameter is end tidal concentration, i.e. the carbon dioxide concentration in the last gas expired by the patient in an expiration. The end tidal concentration of carbon dioxide is indicative of arterial blood gas pressure and, accordingly, shows whether or not the patient is being correctly ventilated. Another parameter related to carbon dioxide output is the minute production of carbon dioxide, normally expressed in an expired volume of carbon dioxide per minute. This parameter is indicative of the patient's general metabolism. Other parameters indicative of the efficacy of patient ventilation are also well-known.

One known carbon dioxide analyzer is described in the Operating Manual for the CO₂ Analyzer 930, AG 0291 2.5, July 1981, Siemens-Elema AB. The known carbon dioxide analyzer is connected to a cuvette on the Y-piece of the tubing system connected to the patient. Carbon dioxide is measured using conventional IR spectrophotometry. The analyzer comprises a light source, a filter and a detector. The filter allows passage of a light wavelength at which carbon dioxide absorbs the light. Since the analyzer is located in the Y-piece, gas passes the gas cuvette in two directions, i.e. during inspiration, when fresh gas is carried from the ventilator unit to the patient, and during expiration, when gas is carried from the patient in an expiratory tube back to the ventilator unit. At the end of the inspiratory phase, the carbon dioxide analyzer is zeroed to obtain a reference level for 0% carbon dioxide. Zeroing is necessary with this kind of analyzer, since the detector signal would otherwise generate erroneous values for the carbon dioxide concentration. The placement of the analyzer on the Y-piece, makes it necessary to position it near the patient, but since it also generates heat during operation it must not come into contact with the patient's skin. A heat shield is also often used to further protect the patient from the hot analyzer. In addition to heat generation, other problems are associated with this arrangement for a carbon dioxide analyzer. As noted above, the analyzer is zeroed in the final phase of inspiration. Gas supplied to the patient is normally dry, and the zero value for carbon dioxide is therefore for dry air. Before the gas is delivered to the patient's lungs, it can pass a humidifier which humidifies the gas. Irrespective of whether a humidifier is used or not, gas expired by the patient is saturated with water. Gas expired by the patient can also contain secretion etc. Since the carbon dioxide concentration is therefore measured from gas saturated with moisture, for which correction must be made in determinations of the carbon dioxide concentration, the deposition of condensation or secretion on the cuvette's windows, or something else preventing the light beam from passing the cuvette unimpeded, is a risk. Increase in the common tube for inspiration and expiration also increases dead space.

Another problem, which could develop when the carbon dioxide meter is used in anaesthetic systems, is that certain anaesthetic devices operate with closed systems which re-use expired gas. Carbon dioxide is admittedly removed from the gas before it is returned to the patient, but inspired gas could still contain small amounts of carbon dioxide, and the carbon dioxide analyzer might therefore be zeroed when the concentration is actually greater than 0%. This would obviously occur also with ventilator systems when ordinary air is supplied to the patient via the ventilator system.

In practice, every manufacturer places the carbon dioxide meter next to the patient, as a matter of principle, so the best possible measurement value is obtained for end tidal concentration. Technical development have accordingly run counter to attempts to minimize and simplify carbon dioxide analyzers, without affecting accuracy, in order to minimize the size of the equipment which needs to be placed near the patient.

In the determination of a plurality of the parameters, such as the volume of expired carbon dioxide, the minute production of carbon dioxide etc., respiratory gas flow is also measured. This is normally performed with a flow meter arranged in the ventilator/anaesthetic unit. However, pressure changes in the expiratory tube can cause compression of the volume, and the measured flow will then fail to correspond to the patient's expired flow. Errors also occur in calculations of the parameter.

One objective of the present invention is to achieve a ventilator/anaesthetic system in which accurate measurement of parameters related to the patient's carbon dioxide output can be performed while the aforementioned problems are simultaneously resolved.

One such ventilator/anaesthetic system is achieved in accordance with the invention in that the carbon dioxide meter is devised to generate a measurement signal and a reference signal, the carbon dioxide concentration then being determinable from the relationship between the measurement signal and the reference signal, the carbon dioxide meter is arranged downstream from the patient tube, near the flow meter, to measure the concentration of carbon dioxide in expired breathing gas, and the calculation unit is adapted to determine the parameter from the values measured for flow and the carbon dioxide concentration in at least two breaths.

US 4 619 269 discloses a ventilator system for monitoring respiratory gases, with a carbon dioxide meter which is calibrated at two points. Such two-point calibration is not necessary with carbon dioxide meters using a reference signal and a measurement signal.

Carbon dioxide meters, which generate a measurement signal and a reference signal, are well-known. They can e.g. be constructed so a filter wheel rotates and alternately interposes different filters in the beam path between the light source and the light detector. One filter then passes a light wavelength at which carbon dioxide absorbes light, and another filter passes a wavelength at which carbon dioxide does not absorb light. Alternately, the number of light detectors can be doubled, and a fixed filter placed in front of the respective detector, each filter passing a specific wavelength. A carbon dioxide meter according to the latter design is disclosed in EP-A-O 584 519. Both types of carbon dioxide meters have the advantage of not needing to be zeroed periodically, since they generate a reference signal at a wavelength at which carbon dioxide does not absorb light. This is necessary for performing the next step in the invention, viz, to move the carbon dioxide meter from the Y-piece to the flow meter itself. This means that all the additional equipment related to carbon dioxide measurement is moved away from the patient, thereby greatly facilitating work for staff around the patient. Dead space will also decrease. The measurement instruments can be arranged in different ways. Both the flow meter and the carbon dioxide meter can advantageously be arranged inside the ventilator/anaesthetic unit.

Another advantage of the new location for the carbon dioxide meter is a reduction in the impact on measurements exerted by the patient's moisture-laden expired air, and any secretion in it. Secretion is collected in a special container near the patient, and expired gas can be dehumidified to a greater or lesser degree in a dehumidifier before it reaches the carbon dioxide meter and the flow meter. In addition, prevention of condensation etc. on the windows of the measurement cuvette is facilitated, since the carbon dioxide meter can easily be heated to a much higher temperature at its new location than is acceptable from the safety point of view when located at the Y-piece.

Moving the carbon dioxide meter from the Y-piece, near the patient, to a position close to, or inside, the ventilator/anaesthetic unit might seem like a relatively simple measure, since a location near the patient does cause some problems. However, there are a number of reasons why this has not been possible before. First of all, the end tidal concentration of carbon dioxide has often been cited as one of the most important carbon dioxide parameters. For correct measurement of this parameter, measurement has been performed as close to the lungs as possible. It must also be remembered that expired gas fills the entire Y-piece and the expiratory tube, like a column of gas, during expiration. The diffusion of gas between breaths obliterates the sharp demarcations at the beginning and end of this gas column. With a system employing continuous flows of passing gas, i.e. bypass flow, existing gas in the patient tube and expiratory tube is indeed expelled, but it then mixes with passing gas, something which does not occur in the common put of the Y-piece.

This therefore makes it impossible to measure the end tidal concentration of carbon dioxide in one and the same breath when the position of the carbon dioxide meter has been changed. It will be realized that any such change in the position of the carbon dioxide meter is by no means self-evident. So a different method for the calculation unit's calculation of the parameters, and the extraction of same from measurement values, has been called for. The invention achieves this in that the calculation unit is devised to determine the parameters from values measured for flow and carbon dioxide concentration in at least two respiratory cycles. When the volume of gas in the expiratory tube and Y-piece and the delay until carbon dioxide measurement are known, diffusion in the column of gas can be determined, and the parameters can be corrected. It should be emphasized that continuous measurement throughout two respiratory cycles is not necessary for determining the parameters. Measurement during parts of the cycles is fully sufficient. Measurement during the measurement periods can be performed in the known manner, e.g. analog (continuous) measurement or digital measurement (with a predefined sampling rate).

A more sensitive carbon dioxide meter can be used when the carbon dioxide meter is placed inside the ventilator/anaesthetic unit.

It is advantageous if the calculation unit comprises an integrator and if the calculation unit is adapted to determine a minute volume of carbon dioxide from the integral of the product of the measured carbon dioxide concentration and flow. Compared to previously known carbon dioxide meters and equipment, a direct product of carbon dioxide concentration and flow can be obtained with a system according to the invention. Since the carbon dioxide meter and flow meter are near each other, the product directly designates the concentration of carbon dioxide in the flow. The integral of the product yields the volume. As noted above, the measurement does not require all the values from two respiratory cycles.

The end tidal concentration, viewed in the art as the most interesting parameter, can be determined in at least two ways by the system according to the invention.

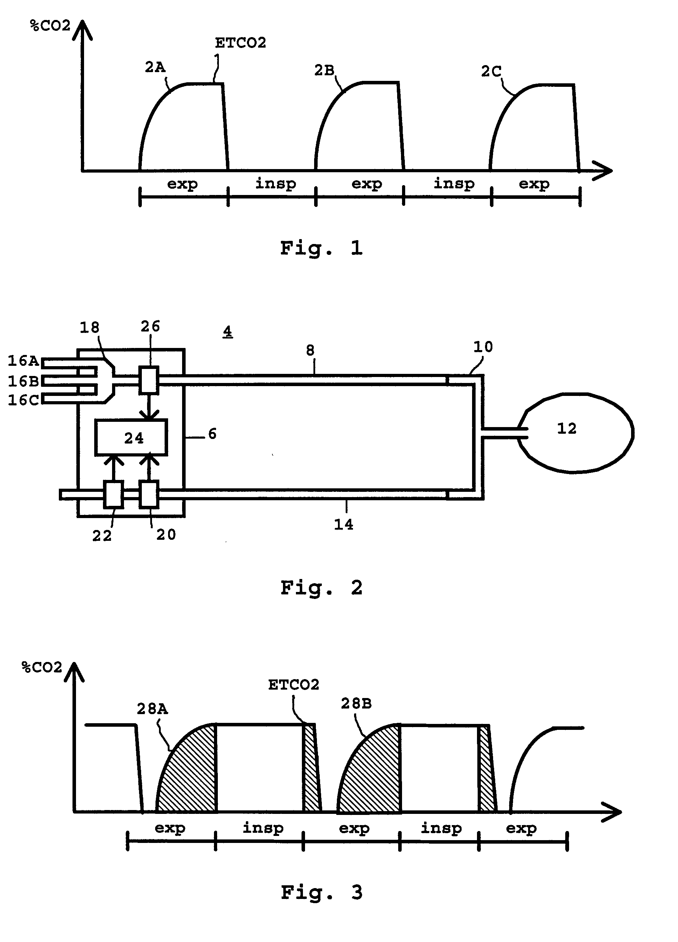

The end tidal concentration of carbon dioxide in one breath can be determined as the peak carbon dioxide concentration, measured by the carbon dioxide meter, in the following breath. In principle, the contents of the expiratory tube and patient tube consist of a column of gas. In the final phase of an expiration, there is therefore a column of expired gas in the expiratory tube and the patient tube. During the next inspiration, fresh breathing gas flushes out part of the patient tube and is supplied to the patient. When the patient again exhales, a smaller column of fresh breathing gas, which does not contain any carbon dioxide, pushes the preceding breath's column of gas ahead of it through the ventilator unit and carbon dioxide meter. Measuring the peak value for carbon dioxide concentration in one expiration yields a good value for the end tidal concentration in the preceding breath. One small difference, compared to previous measurement systems, may develop because of the diffusion of gas between different gas columns and because of mixing effects between gas columns, if any turbulence occurs. However, this is not a serious adverse effect, since the carbon dioxide curve in expiration rises relatively quickly, as shown in FIG. 1, to a level which is maintained throughout the rest of the expiration, so the true end tidal concentration (ETCO₂) does not differ very much, even if there has been some mixture of expired gas and fresh gas. As already noted, diffusion can be determined and calculation of the end tidal concentration can thereby be corrected for that diffusion.

Alternately, the end tidal concentration can be obtained by utilizing the fact that a known volume of expired breathing gas fills the expiratory tube and the patient tube between the carbon dioxide meter and the patient, the calculation unit comprises an integrator for integrating the measurement value from the flow meter and an end tidal concentration of carbon dioxide in one breath is determined as the peak concentration measured by the carbon dioxide meter in the next breath, when a volume corresponding to the known volume has passed the flow meter.

In principle, this method is based on the same reasoning as in the above-described method for obtaining the end tidal concentration. In this instance, however, the known volume is regarded as a unit, and the end tidal concentration is determined at the time the known volume has passed the flow meter.

In practice, the two described methods supply essentially the same value for end tidal concentration. However, the delay between the end tidal concentration for a specific expiration until measurement of same has occurred can exceed one breath, depending on the tidal volume supplied to the patient and the volumes contained in tubes.

An improvement of the ventilator/anaesthetic system is achieved in accordance with the invention in that a continuous flow of breathing gas which flows through the inspiratory tube, the patient tube and the expiratory tube is supplied by the ventilator/anaesthetic system, an additional flow meter is arranged in the ventilator/anaesthetic system, to measure the flow of gas supplied to the inspiratory tube, and connected to the calculation unit and the calculation unit is adapted to correct the parameter's determination from the continuous flow.

Bypass flows occur in different contexts in conjunction with ventilator/anaesthetic systems. For example, a patient, who only requires limited respiratory support in order to breathe, can then breathe, with relative ease, from the passing flow of breathing gas. However, determination of the parameters must be corrected for this continuous flow.

In the case of end tidal concentration, for example, this means that the column of gas which would otherwise have filled the inspiratory tube and part of the patient tube will be expelled more rapidly from the ventilator/anaesthetic system. In addition, this column of gas will mix with fresh gas, thereby affecting measurement of concentration. Since the supplied flow of breathing gas is known, thanks to the additional flow meter, however, the impact of this flow on determination of the parameters can be corrected. The parameter is still determined with measurement values from two respiratory cycles, since the final volume expired by the patient is expelled by the continuous flow following each concluded expiration.

If the volume of the expiratory tube is not known, this volume can be determined by removing the bypass flow for at least one breath and recording when the carbon dioxide value changes. The integral of the value for flow then designates the volume of the tube. Moreover, the curve for the carbon dioxide concentration at the Y-piece can be recreated, with the known tube volume, by correcting for the effect of tube volume.

One embodiment of the invention will now be described in greater detail, referring to the figures in which
FIG. 1 shows the carbon dioxide concentration during a patient's inspiration and expiration respectively;
FIG. 2 shows one embodiment of a ventilator system according to the invention; and
FIG. 3 shows the morphology of a measurement signal from a carbon dioxide meter by the valve system according to the invention.

As also noted above, FIG. 1 shows curves for the concentration of carbon dioxide in air expired by a patient. Here, curves 2A, B, C show how the concentration of carbon dioxide rapidly levels off during expiration (exp). At the end of expiration, the concentration rapidly drops towards zero. The end tidal concentration of carbon dioxide, ETCO₂, is determined at the end of expiration. During inspiration (insp), the concentration is normally equal to zero.

FIG. 2 shows an embodiment of the invention in the form of a ventilator system 4. The ventilator system 4 comprises a ventilator unit 6 from which an inspiratory tube 8 carries a breathing gas, via a patient tube 10, to a patient 12. The patient tube 10 is also referred to as a Y-piece or Y-tube. Expired gas is carried from the patient 12, via the patient tube 10 and an expiratory tube 14, back to the ventilator unit 6. Breathing gas supplied to the patient is admitted via one or more of three gas connections 16A, B, C and mixed in a mixing chamber 18 before being carried to the inspiratory tube 8.

It should be noted that the ventilator unit 6 also comprises a number of other components than those shown in the figure. In principle, the ventilator unit 6 can consist of e.g. a modified Servo Ventilator 300, Siemens-Elema AB. Check valves can be arranged in the respiratory system to control the direction of gas flow in the inspiratory tube 8, patient tube 10 and expiratory tube 14.

Expired gas passes a first flow meter 20, which is arranged in the ventilator unit 6. The flow of expired gas is measured in this flow meter. A carbon dioxide meter 22 is arranged next to the first flow meter 20. The carbon dioxide meter 22 measures the concentration of carbon dioxide in expired gas. In principle, any carbon dioxide meter will suffice, provided the meter generates a measurement signal and a reference signal, the concentration of carbon dioxide being determined from the ratio between the measurement signal and the reference signal. The first flow meter 20 and the carbon dioxide meter 22 are connected to a calculation unit 24, which calculates or determines at least one parameter related to the patient's 12 carbon dioxide output. In the event that a continuous flow of gas is admitted via the inspiratory tube and flows through the patient tube 10 and the expiratory tube 14, a second flow meter 26 is arranged in the ventilator unit 6 to measure the continuous flow. The second flow meter 26 is connected to the calculation unit 24, which can accordingly correct the determination of the parameter, or parameters, for the continuous flow.

In contrast to previously known systems with carbon dioxide meters or carbon dioxide analyzers located in the patient tube 10 near the patient, the calculation unit 24 must be devised to take into account the altered location of the carbon dioxide meter 22. In particular, the fact that there is a given volume of expired gas in the expiratory tube 14 and the patient tube 10 after concluded expiration must be taken into account. This volume of gas does not normally reach the carbon dioxide meter 22 until the next expiration. This is illustrated more clearly in FIG. 3 which shows the measurement signal from the carbon dioxide meter 22 for the expiratory curves shown in FIG. 1. The curve 28A shows that the carbon dioxide meter 22 does not measure gas expired in a breath until some point into the patient's expiration, as shown in the diagram. When the patient terminates an expiration and commences inspiration, the measured concentration of carbon dioxide will remain on a constant level, since gas in the expiratory tube 14 is motionless.

During the next expiration, the volume of gas filling the expiratory tube 14 and part of the patient tube 10 is pushed forward through the carbon dioxide meter 22 and the first flow meter 20. The rest of the gas expired in the preceding breath will then pass the carbon dioxide meter 22, and a value for e.g. the end tidal concentration, ETCO₂, can be determined for the preceding breath. This determination can be performed in such a way that the peak value measured for carbon dioxide in each breath serves as the end tidal concentration of carbon dioxide in the preceding breath. Presentation of the end tidal concentration with a delay of one breath is not a major problem for the physician. If some drastic event were to occur in respect to the patient's 12 output of carbon dioxide, it would most likely be manifest even in the part of the curve measured during the current breath. Such an event could be e.g. the failure of the carbon dioxide meter 22 to measure any carbon dioxide content, even though the patient is exhaling.

Other parameters which could be determined are e.g. effective and ineffective tidal volumes, the minute volume of carbon dioxide in expired gas and the minute production of carbon dioxide by the patient 12. In the corresponding manner as for end tidal concentration, these parameters are determined from information derived from at least two breaths. For example, the minute volume of carbon dioxide in expired gas, which can be determined from the integral of the product of concentration and flow. Then, it does not matter that the concentration measured during an inspiration is consistently high, since the flow is zero, and flow would have no impact on the determination of the minute volume of carbon dioxide, nor in determination of the minute production of carbon dioxide. Tidal volume is obtained in the corresponding manner by e.g. integrating the product of concentration and flow for e.g the concentration curve 28A.

When a continuous flow of breathing gas flows through the tubes 8, 10, 14, the calculation unit 24 must correct the calculated parameters for this continuous flow. The most important difference is found in the determination of end tidal concentration, since measurement of concentration alone is then no longer sufficient. The continuous flow will cause the column of gas, or volume of gas, in the expiratory tube 14 at the end of expiration, according to the reasoning above, to be expelled more rapidly from the system. Since the continuous flow is known from the second flow meter 26, the patient flow can be determined as the difference between flows measured in the first flow meter 20 and in the second flow meter 26. Because of the integration, passing volumes are known, and the concentration figure for the entire passing flow, or volume, can be converted into a concentration for the volume of expired gas.

The ventilator system 4 could also comprise an anaesthetic system according to some prior art design. The salient features of the invention are that measurement of carbon dioxide is made near measurement of flow, and measurement has been transferred to a point downstream from the patient in the direction of expiratory flow.

## Claims

1. A ventilator/anaesthetic system (4) comprising a ventilator/anaesthetic unit (6), an inspiratory tube (8) to carry a breathing gas from the ventilator/anaesthetic unit (6) to a patient (12), an expiratory tube (14) to carry expired breathing gas from the patient (12) to the ventilator/anaesthetic unit (6), a patient tube (10), connected to the inspiratory tube (8) and the expiratory tube (14) and connectable to the patient's (12) airways, a flow meter (20) arranged to measure the flow of expired gas, a carbon dioxide meter (22) to measure the concentration of carbon dioxide in expired breathing gas and a calculation unit (24), connected to the carbon dioxide meter (22) and the flow meter (20), to determine at least one parameter related to the patient's carbon dioxide output, **characterized in that** the carbon dioxide meter (22) is devised to generate a measurement signal and a reference signal, the carbon dioxide concentration then being determinable from the relationship between the measurement signal and the reference signal, the carbon dioxide meter (22) is arranged downstream from the patient tube (10), near the flow meter (20), to measure the concentration of carbon dioxide in expired breathing gas, and the calculation unit (24) is adapted to determine the parameter from the values measured for flow and the carbon dioxide concentration in at least two respiratory cycles.

2. A ventilator/anaesthetic system according to claim 1, **characterized in that** the calculation unit (24) comprises an integrator, and the calculation unit is adapted to determine a minute volume for carbon dioxide from the integral of the product of the measured carbon dioxide concentration and flow.

3. A ventilator/anaesthetic system according to claim 1 or 2, **characterized in that** an end tidal concentration of carbon dioxide in one breath is determinable as the concentration measured by the carbon dioxide meter (24) in the next breath.

4. A ventilator/anaesthetic system according to claim 1 or 2, **characterized in that**, it is utilizing the fact that a known volume of expired breathing gas fills the expiratory tube (8) and the patient tube (10) between the carbon dioxide meter (24) and the patient (12), the calculation unit (24) comprises an integrator for integrating the measurement value from the flow meter (20) and an end tidal concentration of carbon dioxide in one breath is determinable as the peak concentration measured by the carbon dioxide meter (24) in the next breath, after a volume corresponding to the known volume has passed the flow meter (20).

5. A ventilator/anaesthetic system according to any of the above claims, **characterized in that** a continuous flow of breathing gas which flows through the inspiratory tube (8), the patient tube (10) and the expiratory tube (14) can be supplied by the ventilator/anaesthetic unit (6), an additional flow meter (26), arranged in the ventilator/anaesthetic unit (6) to measure the flow of gas supplied to the inspiratory tube (8), is connected. to the calculation unit (24) and the calculation unit (24) is adapted to correct determination of the parameter from the continuous flow.

## Patentansprüche

1. Ein Beatmungsgerät/Narkosesystem (4) mit einer Beatmungs-/Narkoseeinheit (6), einer Inspirationsleitung (8) zum Leiten eines Atemgases von der Beatmungs-/Narkoseeinheit (6) zu einem Patienten (12), einer Exspirationsleitung (14) zum Leiten des ausgeatmeten Atemgases von dem Patienten (12) zu der Beatmungs-/Narkoseeinheit (6), einer Patientenleitung (10), die mit der Inspirationsleitung (8) und der Exspirationsleitung (14) verbunden und mit den Luftwegen des Patienten (12) verbindbar ist, einem zum Messen des Flusses ausgeatmeten Gases vorgesehenen Flussmesser (20), einem Kohlendioxidmesser (22) zum Messen der Konzentration von Kohlendioxid in ausgeatmetem Atemgas und einer Berechnungseinheit (24), die mit dem Kohlendioxidmesser (22) und dem Flussmesser (20) verbunden ist, um zumindest einen mit der Kohlendioxidproduktion des Patienten in Verbindung stehenden Parameter zu bestimmen, **dadurch gekennzeichnet, dass** der Kohlendioxidmesser (22) ausgebildet ist, um ein Messsignal und ein Referenzsignal zu erzeugen, wobei die Kohlendioxidkonzentration dann aus dem Verhältnis zwischen dem Messsignal und dem Referenzsignal bestimmbar ist, der Kohlendioxidmesser (22) hinter der Patientenleitung (10) nahe dem Flussmesser (20) angeordnet ist, um die Konzentration von Kohlendioxid in ausgeatmetem Atemgas zu messen, und die Berechnungseinheit (24) angepasst ist, um den Parameter aus für den Fluss und die Kohlendioxidkonzentration gemessenen Werten in zumindest zwei Atemzyklen zu bestimmen.

2. Ein Beatmungsgerät/Narkosesystem gemäß Anspruch 1, **dadurch gekennzeichnet, dass** die Berechnungseinheit (24) einen Integrator aufweist und die Berechnungseinheit angepasst ist, um ein Minutenvolumen für Kohlendioxid aus dem Integral des Produktes der/des gemessenen Kohlendioxidkonzentration und Flusses zu bestimmen.

3. Ein Beatmungsgerät/Narkosesystem gemäß Anspruch 1 oder 2, **dadurch gekennzeichnet, dass** eine Endtidalkonzentration von Kohlendioxid in einem Atemzug als die in dem nächsten Atemzug von dem Kohlendioxidmesser (24) gemessene Konzentration bestimmbar ist.

4. Ein Beatmungsgerät/Narkosesystem gemäß Anspruch 1 oder 2, **dadurch gekennzeichnet, dass** es die Tatsache ausnutzt, dass ein bekanntes Volumen ausgeatmeten Gases die Exspirationsleitung (8) und die Patientenleitung (10) zwischen dem Kohlendioxidmesser (24) und dem Patienten (12) füllt, die Berechnungseinheit (24) einen Integrator zum Integrieren des Messwertes von dem Flussmesser (20) aufweist und eine Endtidalkonzentration von Kohlendioxid in einem Atemzug als die durch den Kohlendioxidmesser (24) gemessene Spitzenkonzentration in dem nächsten Atemzug, nachdem ein zu dem bekannten Volumen korrespondierendes Volumen den Flussmesser (20) passiert hat, bestimmbar ist.

5. Ein Beatmungsgerät/Narkosesystem gemäß einem der obigen Ansprüche, **dadurch gekennzeichnet, dass** ein kontinuierlicher Fluss von Atemgas, der durch die Inspirationsleitung (8) , die Patientenleitung (10) und die Exspirationsleitung (14) fließt, durch die Beatmungsgerät/Narkoseeinheit (6) zugeführt werden kann, ein zusätzlicher, in der Beatmungsgerät/Narkoseeinheit (6) ) zum Messen des Flusses des der Inspirationsleitung (8) zugeführten Gases angeordneter Flussmesser (26) mit der Berechnungseinheit (24) verbunden ist und die Berechnungseinheit (24) angepasst ist, um die Bestimmung des Parameters aus dem kontinuierlichen Fluss zu korrigieren.

## Revendications

1. Système (4) anesthésique/ventilateur comportant une unité (6) anesthésique/ventilateur, un tube (8) d'inspiration pour acheminer un gaz de respiration de l'unité (6) anesthésique/ventilateur vers un patient (12), un tube (14) d'expiration pour acheminer du gaz de respiration expiré du patient (12) vers l'unité (6) anesthésique/ventilateur, un tube (10) de patient, relié au tube (8) d'inspiration et au tube (14) d'expiration et pouvant être connecté aux voies de respiration du patient (12), un débitmètre (20) disposé pour mesurer le débit de gaz expiré, un dispositif (22) de mesure de dioxyde de carbone pour mesurer la concentration en dioxyde de carbone dans le gaz de respiration expiré et une unité (24) de calcul, connectée au dispositif (22) de mesure de dioxyde de carbone et au débitmètre (20), pour déterminer au moins un paramètre lié à l'émission en sortie de dioxyde de carbone du patient, **caractérisé en ce que** le dispositif (22) de mesure de dioxyde de carbone est conçu pour produire un signal de mesure et un signal de référence, la concentration en dioxyde de carbone étant alors déterminable à partir de la relation entre le signal de mesure et le signal de référence, le dispositif (22) de mesure de dioxyde de carbone étant disposé en aval du tube (10) de patient, à proximité du débitmètre (20), pour mesurer la concentration en dioxyde de carbone dans le gaz de respiration expiré, et l'unité (24) de calcul est conçue pour déterminer le paramètre à partir des valeurs mesurées pour le débit et la concentration en dioxyde de carbone dans au moins deux cycles respiratoires.

2. Système anesthésique/ventilateur suivant la revendication 1, **caractérisé en ce que** l'unité (24) de calcul comporte un intégrateur, et l'unité de calcul est conçue pour déterminer un volume minute pour le dioxyde de carbone à partir de l'intégrale du produit de la concentration en dioxyde de carbone mesurée et du débit.

3. Système anesthésique/ventilateur suivant la revendication 1 ou 2, **caractérisé en ce qu'**une concentration de fin d'expiration en dioxyde de carbone dans une respiration peut être déterminée en tant que la concentration mesurée par le dispositif (24) de mesure de dioxyde de carbone dans la respiration suivante.

4. Système anesthésique/ventilateur suivant la revendication 1 ou 2, **caractérisé en ce qu'**il est utilisé le fait qu'un volume connu de gaz de respiration expiré emplit le tube (8) d'expiration et le tube (10) de patient entre le dispositif (24) de mesure de dioxyde de carbone et le patient (12), l'unité (24) de calcul comporte un intégrateur pour intégrer la valeur de mesure provenant du dispositif (20) de mesure de débit et une concentration de fin d'expiration en dioxyde de carbone dans une respiration peut être déterminée en tant que la concentration pic mesurée par le dispositif (24) de mesure de dioxyde de carbone dans la respiration suivante, après qu'un volume correspondant au volume connu est passé par le débitmètre (20).

5. Système anesthésique/ventilateur suivant l'une quelconque des revendications précédentes, **caractérisé en ce qu'**un débit continu de gaz de respiration qui s'écoule par le tube (8) d'inspiration, le tube (10) de patient et le tube (14) d'expiration peut être fourni par l'unité (6) anesthésique/ventilateur, un débitmètre (26) supplémentaire, disposé dans l'unité (6) anesthésique/ventilateur pour mesurer le débit de gaz fourni au tube (8) d'inspiration, est relié à l'unité (24) de calcul et l'unité (24) de calcul est conçue pour corriger la détermination du paramètre à partir du débit continu.
